# EUROPEAN PATENT APPLICATION

(11) **EP 4 428 252 A1**
(43) Date of publication of application: **11.09.2024**
(21) Application number: 23160763.1
(22) Date of filing: 08.03.2023
(51) Int. Cl.: C12Q 1/689

(54) **OLIGONUCLEOTIDE PRIMERS FOR THE IDENTIFICATION OF A BACTERIUM OF THE GENUS BACILLUS**

(71) Applicant: Kerry Group Services International Limited, Tralee County Kerry V92 EH11 (IE)
(72) Inventor: Vinay, Elena, Oregon, 53575 (US); O'Shea, Eileen, Williams Bay, 53191 (US); Tantika, Ben, Richmond, V7A3V2 (CA); Dimitriu, Pedro, Vancouver, V6E1H8 (CA); Moran, Karl, Dublin, Dublin 16 (IE)
(74) Representative: FRKelly

(57) **Abstract**

The present invention relates to oligonucleotide primers for the identification of a bacterium of the genus Bacillus. Also disclosed are a kit for the identification of Bacillus bacteria, use of the oligonucleotide primers for the identification of Bacillus bacteria, and a method for the identification of Bacillus bacteria.

## Description

### Field of the invention

The present invention relates to oligonucleotide primers for the identification of a bacterium of the genus Bacillus. Also disclosed are a kit for the identification of Bacillus bacteria, use of the oligonucleotide primers for the identification of Bacillus bacteria, and a method for the identification of Bacillus bacteria.

### Background to the invention

Bacillus is a genus of Gram-positive, rod-shaped bacteria and is a member of the phylum Bacillota. Bacillus coagulans is a Bacillus species that was first described in 1915 at the Iowa agricultural experiment station, with regard to the coagulation of canned evaporated milk. Bacillus coagulans GBI-30, 6086 is a Bacillus coagulans strain and is a probiotic organism.

Bacillus coagulans GBI-30, 6086 is L+ lactic acid producing, non-toxicogenic, and non-pathogenic. The strain organism is a Gram-positive, spore-forming rod that is aerobic to microaerophilic in nature, and has a size of about 0.9 microns x 3.0 microns x 5.0 microns.

Bacillus coagulans GBI-30, 6086 is generally recognized as safe (GRAS) for intended uses as an ingredient in baked goods and baking mixes; beverages and beverage bases; breakfast cereals; chewing gum; coffee and tea; condiments and relishes; confections and frostings; dairy product analogs; fruit juices; frozen dairy desserts and mixes; fruit and water ices; gelatins, puddings, and fillings; grain products and pastas; hard candy; herbs, seeds, spices, seasonings, blends, extracts and flavorings; jams and jellies; milk; milk products; nuts and nut products; plant protein products; processed fruits; processed vegetables and vegetable juices; snack foods; soft candy; soups and soup mixes; sugar; and sweet sauces, toppings, and syrups at a maximum level of approximately 2 x 109 colony forming units (CFU) per serving.

Bacillus coagulans GBI-30, 6086 whole-genome sequencing was performed using the Illumina GAllx platform at CRA-Genomics Research Centre (Piacenza, Italy) with a paired-end library, and the sequencing project has been deposited in DDBJ/EMBL/GenBank under the accession number JPSK00000000.

Bacillus coagulans GBI-30, 6086 has been deposited with the American Type Culture Collection (ATCC; Manassus, VA, USA) under the terms of the Budapest Treaty having the ATCC Designation Number PTA-6086 and is the subject of United States Patent No 6,849,256 in the name of GANEDEN BIOTECH INC.

Bacillus coagulans GBI-30, 6086 exhibits digestive and health immune benefits. As the health benefits and dosages for probiotic organisms are strain specific, it is key to have tools for the accurate identification of such strains. There is therefore a need to provide molecular tools for identification of a bacterium of the genus Bacillus, in particular the species strain Bacillus coagulans GBI-30, 6086.

### Summary of the invention

According to a first aspect of the invention there is provided at least one oligonucleotide primer for the identification of a bacterium of the genus Bacillus, wherein the at least one oligonucleotide primer has a nucleic acid sequence selected from:
ACCACTTCCCCAACGGTTTT;
AACCCCTCAAAACAGCCAA;
TCGCTGAGCAGAGAATGAACC;
CGGAAGCTCAGGGTTCTTTT;
TGGGTTTTCATCCATTAGTCG; and
AAGGTGCGGAAGAAGGATGG.

According to a second aspect of the invention there is provided a kit for the identification of a bacterium of the genus Bacillus, the kit comprising instruction for use and at least one oligonucleotide primer having a nucleic acid sequence selected from:
ACCACTTCCCCAACGGTTTT;
AACCCCTCAAAACAGCCAA;
TCGCTGAGCAGAGAATGAACC;
CGGAAGCTCAGGGTTCTTTT;
TGGGTTTTCATCCATTAGTCG; and
AAGGTGCGGAAGAAGGATGG.

According to a third aspect of the claimed invention there is provided use of at least one oligonucleotide primer for the identification of a bacterium of the genus Bacillus, wherein the at least one oligonucleotide primer has a nucleic acid sequence selected from:
ACCACTTCCCCAACGGTTTT;
AACCCCTCAAAACAGCCAA;
TCGCTGAGCAGAGAATGAACC;
CGGAAGCTCAGGGTTCTTTT;
TGGGTTTTCATCCATTAGTCG; and
AAGGTGCGGAAGAAGGATGG.

According to a fourth aspect of the invention there is provided a method for the identification of a bacterium of the genus Bacillus, the method comprising the steps of:
providing a sample to be tested; and
detecting the presence, absence or quantity of the bacterium of the genus Bacillus in the sample using at least one oligonucleotide primer having a nucleic acid sequence selected from:
   ACCACTTCCCCAACGGTTTT;
   AACCCCTCAAAACAGCCAA;
   TCGCTGAGCAGAGAATGAACC;
   CGGAAGCTCAGGGTTCTTTT;
   TGGGTTTTCATCCATTAGTCG; and
   AAGGTGCGGAAGAAGGATGG.

Optionally, the bacterium is of the species Bacillus coagulans.

Optionally, the bacterium is of the species strain Bacillus coagulans hammer.

Optionally, the bacterium is of the species strain Bacillus coagulans GBI-30, 6086.

Preferably, the bacterium is of the species strain Bacillus coagulans GBI-30, 6086.

Optionally, the at least one oligonucleotide primer is a set of oligonucleotide primers.

Optionally, the set of oligonucleotide primers comprises at least one oligonucleotide primer.

Further optionally, the set of oligonucleotide primers comprises at least two, optionally at least three, optionally at least four, optionally at least five, optionally at least six oligonucleotide primers.

Optionally, the set of oligonucleotide primers comprises at least one pair of oligonucleotide primers.

Optionally, the set of oligonucleotide primers comprises at least one pair of oligonucleotide primers is selected from:
ACCACTTCCCCAACGGTTTT and AACCCCTCAAAACAGCCAA;
TCGCTGAGCAGAGAATGAACC and CGGAAGCTCAGGGTTCTTTT; and
TGGGTTTTCATCCATTAGTCG and AAGGTGCGGAAGAAGGATGG.

Optionally, the at least one pair of oligonucleotide primers is ACCACTTCCCCAACGGTTTT and
AACCCCTCAAAACAGCCAA.

Optionally or additionally, the at least one pair of oligonucleotide primers is
TCGCTGAGCAGAGAATGAACC and CGGAAGCTCAGGGTTCTTTT.

Optionally or additionally, the at least one pair of oligonucleotide primers is
TGGGTTTTCATCCATTAGTCG and AAGGTGCGGAAGAAGGATGG.

Preferably, the set of oligonucleotide primers comprises at least one pair of oligonucleotide primers is selected from:
ACCACTTCCCCAACGGTTTT and AACCCCTCAAAACAGCCAA;
TCGCTGAGCAGAGAATGAACC and CGGAAGCTCAGGGTTCTTTT; and
TGGGTTTTCATCCATTAGTCG and AAGGTGCGGAAGAAGGATGG.

Optionally, the at least one oligonucleotide primer or the set of oligonucleotide primers produces a polynucleotide (PCR product) at least 163bp in length. Further optionally, the at least one oligonucleotide primer or the set of oligonucleotide primers produces a polynucleotide (PCR product) 163bp in length. Still further optionally, the at least one oligonucleotide primer or the set of oligonucleotide primers produces a polynucleotide (PCR product) at least 297bp in length. Still further optionally, the at least one oligonucleotide primer or the set of oligonucleotide primers produces a polynucleotide (PCR product) 297bp in length.

Preferably, the at least one oligonucleotide primer or the set of oligonucleotide primers produces a polynucleotide (PCR product) 163bp or 297bp in length.

Optionally, the set of oligonucleotide primers comprises at least one oligonucleotide primer and produces a polynucleotide (PCR product) 163bp or 297bp in length.

Further optionally, the set of oligonucleotide primers comprises at least two, optionally at least three, optionally at least four, optionally at least five, optionally at least six oligonucleotide primers and produces a polynucleotide (PCR product) 163bp or 297bp in length.

Optionally, the set of oligonucleotide primers comprises at least one pair of oligonucleotide primers and produces a polynucleotide (PCR product) 163bp or 297bp in length.

Optionally, the set of oligonucleotide primers comprises at least one pair of oligonucleotide primers and produces a polynucleotide (PCR product) 163bp or 297bp in length and is selected from:
ACCACTTCCCCAACGGTTTT and AACCCCTCAAAACAGCCAA;
TCGCTGAGCAGAGAATGAACC and CGGAAGCTCAGGGTTCTTTT; and
TGGGTTTTCATCCATTAGTCG and AAGGTGCGGAAGAAGGATGG.

Optionally, the at least one pair of oligonucleotide primers is ACCACTTCCCCAACGGTTTT and AACCCCTCAAAACAGCCAA and produces a polynucleotide (PCR product) 297bp in length.

Optionally or additionally, the at least one pair of oligonucleotide primers is TCGCTGAGCAGAGAATGAACC and CGGAAGCTCAGGGTTCTTTT and produces a polynucleotide (PCR product) 163bp in length.

Optionally or additionally, the at least one pair of oligonucleotide primers is TGGGTTTTCATCCATTAGTCG and AAGGTGCGGAAGAAGGATGG and produces a polynucleotide (PCR product) 297bp in length.

Optionally, the method further comprises the step of isolating nucleic acids from the sample.

Further optionally, the method further comprises the step of isolating nucleic acids from the sample prior to the detecting step.

Optionally, the method comprises the steps of:
providing a sample to be tested;
isolating nucleic acids from the sample; and
detecting the presence, absence or quantity of the bacterium of the genus Bacillus in the sample using the at least one oligonucleotide primer.

Optionally, the detecting step comprises the step of amplifying the nucleic acids isolated from the sample.

Optionally, the method comprises the steps of:
providing a sample to be tested;
isolating nucleic acids from the sample;
amplifying the nucleic acids isolated from the sample using the at least one oligonucleotide primer; and
detecting the presence, absence or quantity of the bacterium of the genus Bacillus in the sample.

Preferably, the method comprises the steps of:
providing a sample to be tested;
isolating nucleic acids from the sample;
amplifying the nucleic acids isolated from the sample using the at least one oligonucleotide primer; and
detecting the presence, absence or quantity of the bacterium of the genus Bacillus in the sample.

Optionally, the presence, absence or quantity of the nucleic acids isolated from the sample using the at least one oligonucleotide primer correlates with the presence, absence or quantity of the bacterium of the genus Bacillus in the sample.

Optionally, the presence, absence or quantity of the nucleic acids isolated from the sample using the at least one oligonucleotide primer is attributable to the presence, absence or quantity of the bacterium of the genus Bacillus in the sample.

Optionally, the presence, absence or quantity of the nucleic acids isolated from the sample using the at least one oligonucleotide primer is proportionate to the presence, absence or quantity of the bacterium of the genus Bacillus in the sample.

Further optionally, the presence, absence or quantity of the nucleic acids isolated from the sample using the at least one oligonucleotide primer is directly proportionate to the presence, absence or quantity of the bacterium of the genus Bacillus in the sample.

Optionally, the amplifying step comprises amplifying the nucleic acids isolated from the sample by polymerase chain reaction.

Optionally, the amplifying step comprises amplifying the nucleic acids isolated from the sample by polymerase chain reaction comprising at least one thermal cycle.

Optionally, the polymerase chain reaction comprises a plurality of thermal cycles.

Optionally, the polymerase chain reaction comprises at least 10 thermal cycles.

Further optionally, the polymerase chain reaction comprises at least 15, optionally at least 20, optionally at least 25, optionally at least 30, optionally at least 35, optionally at least 40, optionally at least 45 thermal cycles.

Preferably, the polymerase chain reaction comprises at least 30 thermal cycles.

Optionally, each thermal cycle comprises a denaturation step, an annealing step, and an elongation step.

Optionally, each denaturation step is conducted at a temperature of at least 94°C.

Further optionally, each denaturation step is conducted at a temperature of at least 95, optionally at least 96, optionally at least 97, optionally at least 98, optionally at least 99°C.

Preferably, each denaturation step is conducted at a temperature of at least 98°C.

Optionally, each annealing step is conducted at a temperature of at least 50°C.

Further optionally, each annealing step is conducted at a temperature of at least 52, optionally at least 54, optionally at least 56, optionally at least 58, optionally at least 60°C.

Preferably, each annealing step is conducted at a temperature of at least 58°C.

Optionally, each elongation step is conducted at a temperature of at least 70°C.

Further optionally, each elongation step is conducted at a temperature of at least 72, optionally at least 74, optionally at least 76, optionally at least 78, optionally at least 80°C.

Preferably, each elongation step is conducted at a temperature of at least 72°C.

Optionally, each denaturation step is conducted for a period of at least 5 seconds.

Further optionally, each denaturation step is conducted for a period of at least 10, optionally at least 15, optionally at least 20, optionally at least 25, optionally at least 30 seconds.

Preferably, each denaturation step is conducted for a period of at least 10 seconds.

Optionally, each annealing step is conducted for a period of at least 15 seconds.

Further optionally, each annealing step is conducted for a period of at least 20, optionally at least 25, optionally at least 30, optionally at least 35, optionally at least 40 seconds.

Preferably, each annealing step is conducted for a period of at least 30 seconds.

Optionally, each elongation step is conducted for a period of at least 15 seconds.

Further optionally, each elongation step is conducted for a period of at least 20, optionally at least 25, optionally at least 30, optionally at least 35, optionally at least 40 seconds.

Preferably, each elongation step is conducted for a period of at least 30 seconds.

Preferably, each denaturation step is conducted at a temperature of at least 98°C for a period of at least 10 seconds.

Preferably, each annealing step is conducted at a temperature of at least 58°C for a period of at least 30 seconds.

Preferably, each elongation step is conducted at a temperature of at least 72°C for a period of at least 30 seconds.

Preferably, the polymerase chain reaction comprises at least 30 thermal cycles, each thermal cycle comprising a denaturation step conducted at a temperature of at least 98°C for a period of at least 10 seconds, an annealing step conducted at a temperature of at least 58°C for a period of at least 30 seconds, and an elongation step is conducted at a temperature of at least 72°C for a period of at least 30 seconds.

Optionally, the polymerase chain reaction further comprises an initialization step.

Optionally, the initialization step is conducted prior to the at least one thermal cycle.

Optionally, the initialization step is conducted at a temperature of at least 94°C.

Further optionally, the initialization step is conducted at a temperature of at least 95, optionally at least 96, optionally at least 97, optionally at least 98, optionally at least 99°C.

Preferably, the initialization step is conducted at a temperature of at least 98°C.

Optionally, the initialization step is conducted for a period of at least 15 seconds.

Further optionally, the initialization step is conducted for a period of at least 30, optionally at least 45, optionally at least 60, optionally at least 120, optionally at least 180, optionally at least 240, optionally at least 300, optionally at least 600 seconds.

Preferably, the initialization step is conducted for a period of at least 30 seconds.

Preferably, the initialization step is conducted at a temperature of at least 98°C for a period of at least 30 seconds.

Optionally, the polymerase chain reaction further comprises a final elongation step.

Optionally, the final elongation step is conducted following the at least one thermal cycle.

Optionally, the final elongation step is conducted at a temperature of at least 70°C.

Further optionally, the final elongation step is conducted at a temperature of at least 71, optionally at least 72, optionally at least 73, optionally at least 74°C.

Preferably, the final elongation step is conducted at a temperature of at least 72°C.

Optionally, the final elongation step is conducted for a period of at least 5 minutes.

Further optionally, the final elongation step is conducted for a period of at least 10, optionally at least 15, optionally at least 20 minutes.

Preferably, the final elongation step is conducted for a period of at least 10 minutes.

Preferably, the final elongation step is conducted at a temperature of at least 72°C for a period of at least 10 minutes.

Optionally, the polymerase chain reaction further comprises a final hold step.

Optionally, the final hold step is conducted following the final elongation step.

Optionally, the final hold step is conducted at a temperature of at least 2°C.

Further optionally, the final hold step is conducted at a temperature of at least 4, optionally at least 6, optionally at least 8, optionally at least 10, optionally at least 12, optionally at least 14, optionally at least 16°C.

Preferably, the final hold step is conducted at a temperature of at least 4°C.

Optionally, the final hold step is conducted for a period of at least 5 minutes.

Further optionally, the final hold step is conducted for a period of at least 30, optionally at least 60, optionally at least 120 minutes, optionally at least 240 minutes, optionally at least 480 minutes, optionally at least 720 minutes, optionally at least 1440 minutes.

Preferably, the final hold step is conducted for a period of at least 5 minutes.

Preferably, the final hold step is conducted at a temperature of at least 4°C for a period of at least 5 minutes.

Optionally, the polymerase chain reaction comprises:
an initialization step; and
at least one thermal cycle, each thermal cycle comprising a denaturation step, an annealing step, and an elongation step.

Optionally, the polymerase chain reaction comprises:
at least one thermal cycle, each thermal cycle comprising a denaturation step, an annealing step, and an elongation step; and
a final elongation step.

Optionally, the polymerase chain reaction comprises:
an initialization step; and
at least one thermal cycle, each thermal cycle comprising a denaturation step, an annealing step, and an elongation step; and
a final elongation step.

Optionally, the polymerase chain reaction comprises:
an initialization step; and
at least one thermal cycle, each thermal cycle comprising a denaturation step, an annealing step, and an elongation step;
a final elongation step; and
a final hold step.

Preferably, the polymerase chain reaction comprises:
an initialization step conducted at a temperature of at least 98°C for a period of at least 30 seconds;
at least 30 thermal cycles, each thermal cycle comprising a denaturation step conducted at a temperature of at least 98°C for a period of at least 10 seconds, an annealing step conducted at a temperature of at least 58°C for a period of at least 30 seconds, and an elongation step is conducted at a temperature of at least 72°C for a period of at least 30 seconds;
a final elongation step conducted at a temperature of at least 72°C for a period of at least 10 minutes; and
a final hold step conducted at a temperature of at least 4°C for a period of at least 5 minutes.

### Brief description of the drawings

The invention is described with reference to the accompanying drawings in which:

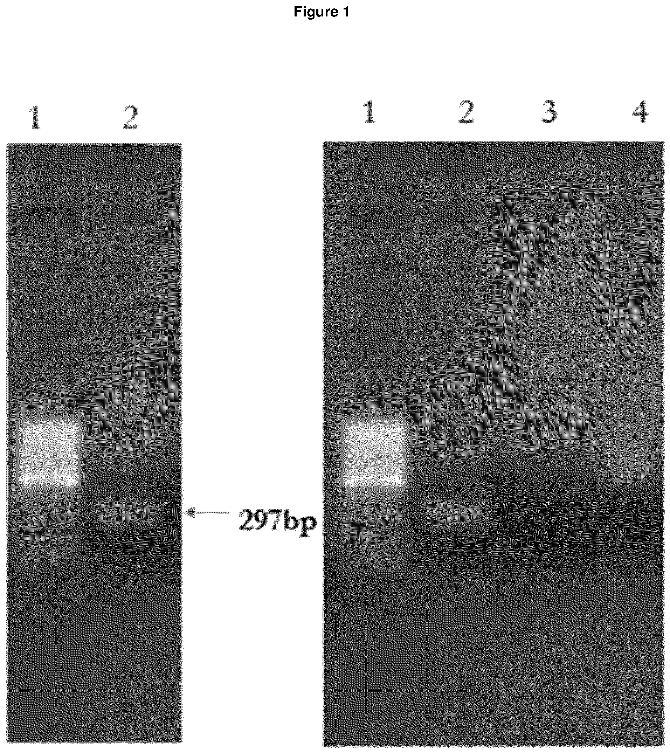

**Figure 1** is an agarose gel indicating PCR product amplification (297bp) using a first primer set with template DNA isolated from Bacillus coagulans GBI-30, 6086 (lane 2) and no amplification product using template DNA isolated from Bacillus coagulans ATCC 7050 (lane 3), wherein nuclease-free sterile water was used as template for negative control (lane 4), and 100 bp DNA ladder was used as reference (lane 1);

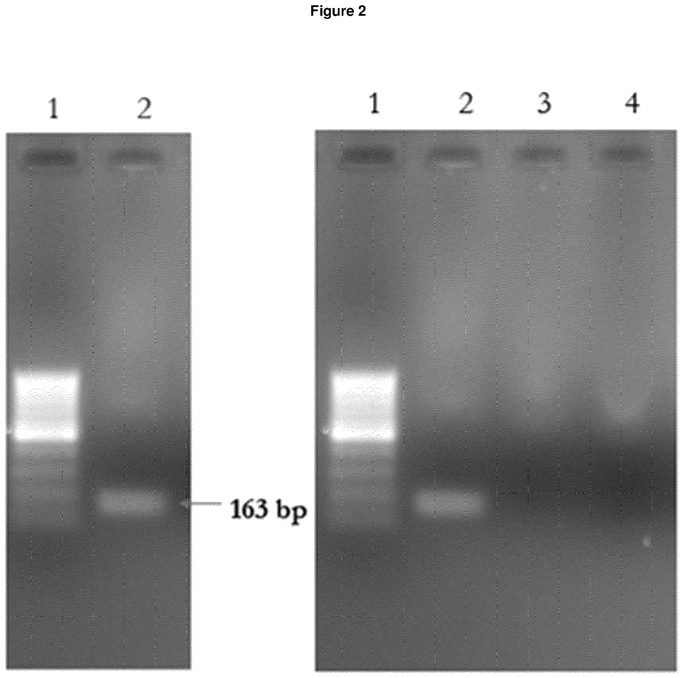

**Figure 2** is an agarose gel indicating PCR product amplification (163bp) using a second primer set with template DNA isolated from Bacillus coagulans GBI-30, 6086 (lane 2) and no amplification product using template DNA isolated from Bacillus coagulans ATCC 7050 (lane 3), wherein nuclease-free sterile water was used as template for negative control (lane 4), and 100 bp DNA ladder was used as reference (lane 1); and

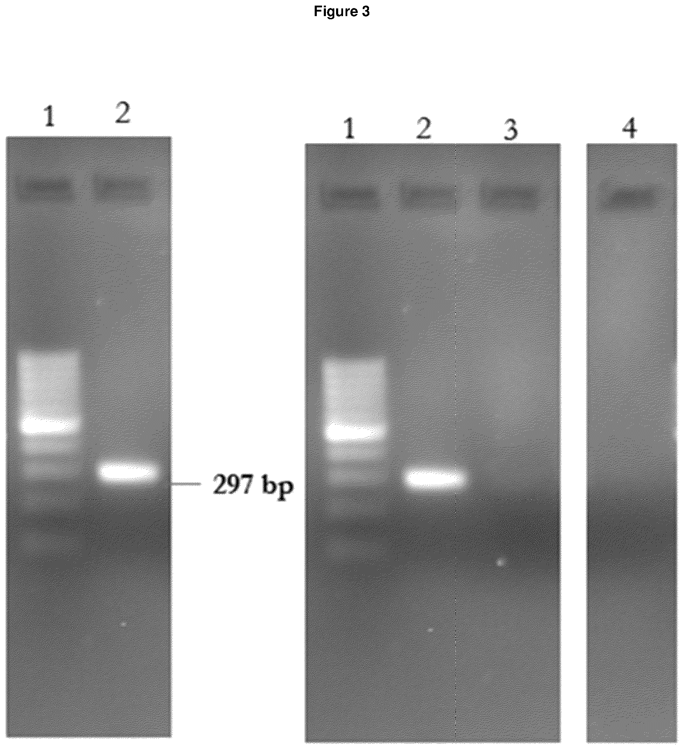

**Figure 3** is an agarose gel indicating PCR product amplification (297bp) using a third primer set with template DNA isolated from Bacillus coagulans GBI-30, 6086 (lane 2) and no amplification product using template DNA isolated from Bacillus coagulans ATCC 7050 (lane 3), wherein nuclease-free sterile water was used as template for negative control (lane 4), and 100 bp DNA ladder was used as reference (lane 1).

### Example

The invention is demonstrated with reference to the following non-limiting example.

### Materials and Methods

### Bacterial strains

Bacillus coagulans GBI-30, 6086 (BC30) is a proprietary spore-forming probiotic and was obtained from the American Type Culture Collection (ATCC) Designation Number PTA-6086.

An additional test strain, Bacillus *coagulans* type strain ATCC 7050, was obtained from the ATCC was used to confirmed specificity of biomarkers.

### Primer design and synthesis

Unique regions of Bacillus coagulans GBI-30, 6086 genomic DNA were targeted for primer design. Sequences of the primers are listed in Table 1. Invitrogen synthesized the primers at a concentration of 25 nanomole. Primers were diluted in nuclease-free sterile water to a stock concentration of 100 micromolar, then further diluted to 10 micromolar in nuclease-free sterile water and stored at -20°C.

### DNA extraction and sample preparation

The Wizard Genomic DNA Purification Kit (Promega) was used to extract DNA from each Bacillus coagulans strain. Manufacturer's specification for Gram-positive bacteria was followed for the extraction. Briefly, cells were resuspended in 50 millimolar EDTA and treated with lysozyme (1mg/mL) for 30-60 min at 37°C. Culture was centrifuged for 2 minutes at 16,000 x g and supernatant removed. Cells were lysed by using the nuclei lysis solutions provided. 600 microliter were added to the pellet and the solution incubated at 80°C for 5 minutes, followed by an RNase treatment. Protein was precipitated using the protein precipitation solution provided, 200 microliter were added to the solution and incubated on ice for 5 minutes. Solution was centrifuged and supernatant used to precipitate DNA with isopropanol (50% v/v) and DNA was dehydrated with the rehydration solution provided overnight at 4°C.

### PCR reactions

PCR amplification was performed on each sample preparation and nuclease-free sterile water was used as the PCR negative control. PCR reactions were preformed using Phusion high-fidelity enzyme PCR master mix with HF Buffer (from New England BioLabs). 25 microliter reactions were followed for reaction set up as followed: 12.5 microliter of 2X Phusion Master Mix, 1.25 microliter of 10 micromolar of forward and 1.25 microliter of 10 micromolar reverse primer, 0.75 microliter DMSO, 1 microliter DNA template and 8.25 microliter of water per reaction were used.

Using a T100 thermal cycler (BIO RAD), the reaction was performed under the following conditions: initialization of 98°C for 30 seconds; followed by 30 cycles of: 98°C for 10 seconds, 58°C for 30 seconds and 72°C for 30 seconds; final elongation of 72°C for 10 minutes; and final hold at 4°C until analysis.

Preparation of a PCR negative control was done replacing the 1 microliter of template DNA sample with 1 microliter of water.

Analysis and visualization of the products of the PCR amplification for each PCR sample preparation and for the PCR negative control was performed using gel electrophoresis. A 100bp DNA ladder standard is used in the first and last lane for determination of amplification product size.

**Table 1. Primer sets for identification of Bacillus coagulans GBI-30, 6086**

| **Primer Sequence (5'-3')** | **Primer** | **SEQ ID NO** | **Set No** | **Product Size** |
|---|---|---|---|---|
| ACCACTTCCCCAACGGTTTT | Forward | 1 | 1 | 297bp |
| AACCCCTCAAAACAGCCAA | Reverse | 2 | | |
| TCGCTGAGCAGAGAATGAACC | Forward | 3 | 2 | 163bp |
| CGGAAGCTCAGGGTTCTTTT | Reverse | 4 | | |
| TGGGTTTTCATCCATTAGTCG | Forward | 5 | 3 | 297bp |
| AAGGTGCGGAAGAAGGATGG | Reverse | 6 | | |

### Results

To confirm specificity of primer sets 1-3 for Bacillus coagulans GBI-30, 6086 (BC30), PCR was performed using template DNA isolated from Bacillus coagulans GBI-30, 6086, and additional test Bacillus coagulans Type strain ATCC 7050, obtained from the American Type Culture Collection (ATCC).

As seen in Figure 1, using Primer Set 1, ACCACTTCCCCAACGGTTTT and AACCCCTCAAAACAGCCAA, a PCR product of 297bp is amplified with template DNA isolated from Bacillus coagulans GBI-30, 6086 (lane 2), but no amplification product is obtained using template DNA isolated from Bacillus coagulans ATCC 7050 (lane 3).

As seen in Figure 2, using Primer Set 2, TCGCTGAGCAGAGAATGAACC and CGGAAGCTCAGGGTTCTTTT, a PCR product of 163bp is amplified with template DNA isolated from Bacillus coagulans GBI-30, 6086 (lane 2) but no amplification product is obtained using template DNA isolated from Bacillus coagulans ATCC 7050 (lane 3).

As seen in Figure 3, using Primer Set 3, TGGGTTTTCATCCATTAGTCG and AAGGTGCGGAAGAAGGATGG, a PCR product of 297bp is amplified with template DNA isolated from Bacillus coagulans GBI-30, 6086 (lane 2) but no amplification product is obtained using template DNA isolated from Bacillus coagulans ATCC 7050 (lane 3).

The results presented demonstrate that each of Primer Sets 1-3 amplify Bacillus coagulans GBI-30, 6086 specific sequence giving a positive PCR result while the reference Bacillus coagulans ATCC7050 strain gave a negative result for each primer set.

## Claims

1. An oligonucleotide primer for the identification of a bacterium of the genus Bacillus, wherein the oligonucleotide primer has a nucleic acid sequence selected from any one of SEQ ID NOs: 1-6.

2. A kit for the identification of a bacterium of the genus Bacillus, the kit comprising instruction for use and at least one oligonucleotide primer according to claim 1.

3. Use of at least one oligonucleotide primer according to claim 1 for the identification of a bacterium of the genus Bacillus.

4. A method for the identification of a bacterium of the genus Bacillus, the method comprising the steps of:
(a) providing a sample to be tested; and
(b) detecting the presence, absence or quantity of the bacterium of the genus Bacillus in the sample using at least one oligonucleotide primer according to claim 1.

5. An oligonucleotide primer according to claim 1, a kit according to claim 2, use according to claim 3, or a method according to claim 4, wherein the bacterium is the species strain Bacillus coagulans GBI-30, 6086.

6. An oligonucleotide primer according to claim 1, a kit according to claim 2, use according to claim 3, or a method according to claim 4, wherein the oligonucleotide primer is a set of oligonucleotide primers comprising at least one pair of oligonucleotide primers selected from:
SEQ ID NO: 1 and SEQ ID NO: 2;
SEQ ID NO: 3 and SEQ ID NO: 4; and
SEQ ID NO: 5 and SEQ ID NO: 6.

7. An oligonucleotide primer, a kit, use, or a method according to claim 6, wherein:
(a) the at least one pair of oligonucleotide primers selected from SEQ ID NO: 1 and SEQ ID NO: 2 produces a polynucleotide (PCR product) 297bp in length;
(b) the at least one pair of oligonucleotide primers selected from SEQ ID NO: 3 and SEQ ID NO: 4 produces a polynucleotide (PCR product) 163bp in length; and/or
(c) the at least one pair of oligonucleotide primers selected from SEQ ID NO: 5 and SEQ ID NO: 6 produces a polynucleotide (PCR product) 297bp in length.

8. A method according to any one of claims 1 and 4-7, wherein the detecting step comprises amplifying nucleic acids isolated from the sample by polymerase chain reaction comprising at least one thermal cycle, wherein each thermal cycle comprises a denaturation step, an annealing step, and an elongation step.

9. A method according to claim 8, wherein the polymerase chain reaction comprises at least 30 thermal cycles.

10. A method according to claim 8 or 9, wherein each denaturation step is conducted at a temperature of at least 98°C for a period of at least 10 seconds.

11. A method according to any one of claims 8-10, wherein each annealing step is conducted at a temperature of at least 58°C for a period of at least 30 seconds.

12. A method according to any one of claims 8-11, wherein each elongation step is conducted at a temperature of at least 72°C for a period of at least 30 seconds.

13. A method according to any one of claims 1 and 4-12, wherein the polymerase chain reaction further comprises an initialization step prior to the at least one thermal cycle, wherein the initialization step is conducted at a temperature of at least 98°C for a period of at least 30 seconds.

14. A method according to any one of claims 1 and 4-13, wherein the polymerase chain reaction further comprises a final elongation step following the at least one thermal cycle, wherein the final elongation step is conducted at a temperature of at least 72°C for a period of at least 10 minutes.

15. A method according to claim any one of claims 1 and 4-14, wherein the polymerase chain reaction further comprises a final hold step following the final elongation step, wherein the final hold step is conducted at a temperature of at least 4°C.
